(19) Europäisches Patentamt
European Patent Office
Office européen des brevets

(11) **EP 2 427 421 B1**

(12) **EUROPÄISCHE PATENTSCHRIFT**

(45) Veröffentlichungstag und Bekanntmachung des Hinweises auf die Patenterteilung:
**16.05.2018 Patentblatt 2018/20**

(21) Anmeldenummer: **10712045.3**

(22) Anmeldetag: **26.03.2010**

(51) Int Cl.:
*C07C 67/54* *(2006.01)*          *C07C 69/54* *(2006.01)*
*B01D 3/12* *(2006.01)*          *B01D 3/42* *(2006.01)*

(86) Internationale Anmeldenummer:
**PCT/EP2010/053963**

(87) Internationale Veröffentlichungsnummer:
**WO 2010/127909 (11.11.2010 Gazette 2010/45)**

(54) **VERFAHREN ZUR AUFREINIGUNG VON MONOMEREN**

METHOD FOR PURIFYING MONOMERS

PROCÉDÉ DESTINÉ À LA PURIFICATION DE MONOMÈRES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **06.05.2009 DE 102009002861**

(43) Veröffentlichungstag der Anmeldung:
**14.03.2012 Patentblatt 2012/11**

(73) Patentinhaber: **Evonik Röhm GmbH**
**64293 Darmstadt (DE)**

(72) Erfinder:
• **BRÖLL, Dirk**
**63225 Langen (DE)**
• **MAUL, Christian**
**67435 Neustadt a. d. W. (DE)**
• **LAUX, Benedikt**
**55234 Monzernheim (DE)**
• **SCHLEEP, Volker**
**64683 Einhausen (DE)**

(56) Entgegenhaltungen:
**EP-A1- 0 365 777     EP-A2- 1 090 904**
**JP-A- 2008 297 242**

• **JAN CVENGROS, ALEXANDER TKAC: "Continuous Processes in Wiped Films. 2. Distilling Capacity and Separating Efficiency of a Molecular Evaporator with a Convex Evaporating Surface" INDUSTRIAL & ENGINEERING CHEMISTRY PROCESS DESIGN AND DEVELOPMENT, Bd. 17, Nr. 3, Juli 1978 (1978-07), Seiten 246-251, XP002593163 DOI: 10.1021/i260067a006**

**Beschreibung**

Die vorliegende Erfindung betrifft ein Verfahren zur Aufreinigung von Monomeren. Darüber hinaus beschreibt die vorliegende Druckschrift eine Anlage zur Durchführung dieses Verfahrens.

Monomere sind reaktive Verbindungen, die leicht polymerisieren können. Zwar kann diese Polymerisation durch Zugabe von Polymerisationsinhibitoren vielfach minimiert werden, jedoch können diese Verbindungen zu Verfärbungen und Nebenreaktionen führen. Weiterhin können größere Stabilisatormengen die Weiterverarbeitung der Monomeren in nachfolgenden Polymerisationsreaktionen nachteilig beeinflussen, so dass einige Spezifikationen den Gehalt an Stabilisatoren begrenzen. Daher stellt die Aufreinigung von Monomeren ein dauerhaftes Problem dar. Dies gilt insbesondere für Monomere mit einem hohen Siedepunkt, da diese bei Bedingungen aufgereinigt werden, bei denen eine Polymerisation nur relativ schwer, d.h. mit hohen Mengen an Polymerisationsinhibitoren unterdrückt werden kann. Dementsprechend ist es äußerst schwierig insbesondere sehr reaktive Monomere mit einem hohen Siedepunkt ohne hohe Ausbeuteverluste in einer sehr hohen Reinheit zu erhalten. Zu diesen Monomeren gehören beispielsweise Hydroxyalkyl(meth)acrylate.

Ein bevorzugtes Verfahren zur Herstellung und Aufreinigung von Hydroxyalkyl(meth)acrylaten wird unter anderem in der europäischen Patentanmeldung EP-A-1 090 904 beschrieben. Demgemäß kann eine Reaktionsmischung, die Hydroxyalkyl(meth)acrylate umfasst, besonders effizient durch eine Destillation, die mit einem Dünnschichtverdampfer kombiniert wird, aufgereinigt werden. Die in EP-A-1 090 904 dargelegten Verfahren führen bei hohen Ausbeuten zu relativ reinen Produkten. Allerdings werden für spezielle Anwendungen besonders reine Hydroxyalkyl(meth)acrylate benötigt. So werden diese Monomere beispielsweise zur Herstellung von Kontaktlinsen eingesetzt, wie dies beispielsweise in US 4,375,534 dargelegt ist. Die hierfür notwendige besonders hohe Produktreinheit kann mit dem in EP-A-1 090 904 dargelegten Verfahren nicht erzielt werden.

EP 0365777 offenbart ein Verfahren zur Herstellung von hochsiedenden Acrylaten und Methacrylaten durch Umesterung mit mehrwertigen Alkoholen, wobei nach der Abtrennung des Katalysators das Produkt einer Kurzwegverdampfung bei einer Sumpftemperatur von 110 bis 170°C und einem Druck von 0,01 bis 10 mbar zuführt. JP 2008297242 beansprucht ein Verfahren zur Herstellung von Alkyladamentyl(meth)acrylat. In einem 2-stufigen Reinigungsprozess werden nach der Entfernung der Verunreinigungen die einen höheren Siedepunkt als Alkyladamantyl(meth)acrylat die Verunreinigungen mit niedrigerem Siedepunkt entfernt.

Die Massenstromdichte der Brüden ist weder in EP 0365777 noch in JP 2008297242 offenbart.

In Anbetracht des Standes der Technik war es nun Aufgabe der vorliegenden Erfindung, ein Verfahren zur Aufreinigung von Monomeren bereitzustellen, mit dem eine besonders hohe Reinheit erhältlich ist. Hierbei sollte das Produkt möglichst in hohen Ausbeuten und, insgesamt gesehen, unter geringem Energieverbrauch erhalten werden. Hierbei sollte die Konzentration an Stabilisator möglichst gering gehalten werden können.

Weiterhin war es mithin Aufgabe der vorliegenden Erfindung ein Verfahren bereitzustellen, mit dem hochsiedende Monomere sicher und einfach aufgereinigt werden können. Hierbei sollte insbesondere auf die Verwendung von großen Mengen an Polymerisationsinhibitoren verzichtet werden können. Darüber hinaus sollten die aufgereinigten Monomere besonders geringe Mengen an Polymerisationsinhibitoren aufweisen, so dass die Inhibierung der Monomerzusammensetzungen auf spezifische Anforderungen, die durch die Weiterverarbeitung gegeben sind, angepasst werden kann.

Gelöst werden diese sowie weitere nicht explizit genannte Aufgaben, die jedoch aus den hierin einleitend diskutierten Zusammenhängen ohne Weiteres ableitbar oder erschließbar sind, durch ein Verfahren mit allen Merkmalen des Patentanspruchs 1. Zweckmäßige Abwandlungen des erfindungsgemäßen Verfahrens werden in den auf Anspruch 1 rückbezogenen abhängigen Ansprüchen unter Schutz gestellt.

Gegenstand der vorliegenden Erfindung ist dementsprechend ein Verfahren zur Aufreinigung von Monomeren, wobei die Monomere (Meth)acrylate mit einer Hydroxygruppe im Alkylrest sind, wobei mindestens ein Teil der in einer Ausgangszusammensetzung enthaltenen Monomere verdampft und anschließend kondensiert wird, welches dadurch gekennzeichnet ist, dass mindestens ein Teil der Ausgangszusammensetzung in einem Kurzwegverdampfer verdampft wird, wobei die Massenstromdichte der Brüden $\dot{m}$ gemäß der Relation (I)

$$\dot{m} \leq 1800 \frac{kg \cdot \sqrt{K}}{mbar \cdot m^2 \cdot h \cdot \sqrt{\dfrac{kg}{kmol}}} \cdot p_i \cdot \left(\frac{\widetilde{M}}{T}\right)^{0,5} \tag{I},$$

worin

$\tilde{M}$ die durchschnittliche Molmasse der Brüden im Kurzwegverdampfer in kg/kmol

T Temperatur der Brüden in K

$p_i$ Druck im Kurzwegverdampfer in mbar

ṁ die Massenstromdichte der Brüden in kg/(m²·h) ist, gewählt wird und

die Ausgangszusammensetzung mindestens 95 Gew.-% Monomer umfasst. Hierdurch gelingt es auf nicht vorhersehbare Weise ein Verfahren zur Aufreinigung von Monomeren bereitzustellen, mit dem eine besonders hohe Reinheit erzielt werden kann. Hierbei kann das Produkt in hohen Ausbeuten und, insgesamt gesehen, unter geringem Energieverbrauch erhalten werden. Durch das erfindungsgemäße Verfahren können insbesondere hochsiedende Monomere sicher und einfach aufgereinigt werden. Hierbei kann insbesondere auf die Verwendung von großen Mengen an Polymerisationsinhibitoren verzichtet werden. Darüber hinaus weisen die aufgereinigten Monomere besonders geringe Mengen an Polymerisationsinhibitoren auf, so dass die Inhibierung der Monomerzusammensetzungen auf spezifische Anforderungen angepasst werden kann.

Darüber hinaus beschreibt die vorliegende Druckschrift eine Anlage zur Durchführung des Verfahrens. Diese Anlage kann kostengünstig hergestellt und betrieben werden. Insbesondere zeichnet sich die Anlage durch lange Wartungsintervalle, kurze Stillstandszeiten und eine einfache Steuerung aus. Erfindungsgemäß wird mindestens ein Teil einer Ausgangszusammensetzung, enthaltend mindestens ein Monomer, verdampft und anschließend kondensiert. Von besonderem Interesse sind hierbei insbesondere Verfahren, bei denen bevorzugt 5 bis 95 Gew.-%, besonders bevorzugt 10 bis 80 Gew.-% und ganz besonders bevorzugt 20 bis 60 Gew.-% der Ausgangszusammensetzung verdampft werden. Bei kontinuierlichen Verfahren ergibt sich diese Größe aus den Gewichtsanteilen des aufgereinigten Monomers zu den nicht verdampften Anteilen der Ausgangszusammensetzung, die aus dem Verdampfer ausgeschleust werden.

Der Begriff "Ausgangszusammensetzung" bezeichnet in diesem Zusammenhang die Zusammensetzung, die in den Kurzwegverdampfer eingeleitet wird, um die Monomere zu verdampfen und zu kondensieren.

Monomere sind vorliegend radikalisch polymerisierbare Verbindungen, die mindestens eine Kohlenstoff-Kohlenstoff-Doppelbindung aufweisen, die vorzugsweise endständig ist.

Zu diesen Monomeren gehören insbesondere Monomere mit einer Säuregruppe, Estergruppen umfassenden Monomere und Styrolmonomere.

Zu den bevorzugten Estergruppen umfassenden Monomeren gehören insbesondere (Meth)acrylate, Fumarate, Maleate und/oder Vinylacetat. Der Ausdruck (Meth)acrylate umfasst Methacrylate und Acrylate sowie Mischungen aus beiden. Diese Monomere sind weithin bekannt.

Erfindungsgemäß eingesetzte Monomere sind (Meth)acrylate mit einer Hydroxygruppe im Alkylrest, insbesondere 2-Hydroxyethylmethacrylat (HEMA), 2-Hydroxyethylacrylat, Hydroxypropylmethacrylat, insbesondere 2-Hydroxypropylmethacrylat und 3-Hydroxypropylmethacrylat (HPMA), und/oder Hydroxypropylacrylat, insbesondere 2-Hydroxypropylacrylat und 3-Hydroxypropylacrylat, Hydroxybutyl(meth)acrylat, vorzugsweise Hydroxybutylmethacrylat (HBMA), 2,3-Dihydroxypropyl(meth)acrylat 3,4-Dihydroxybutyl(meth)acrylat,

(Meth)acrylate, die sich von ungesättigten Fettsäuren oder Fettsäureamiden ableiten, wie (Meth)acryloyloxy-2-hydroxypropyl-linolsäureester, (Meth)acryloyloxy-2-hydroxypropyl-linolensäureester, (Meth)acryloyloxy-2-hydroxypropyl-ölsäureester. Mit überraschenden Vorteilen kann das vorliegende Verfahren insbesondere zur Aufreinigung von hochsiedenden Monomeren eingesetzt werden. Unter hochsiedenden Monomeren werden vorliegend Monomere mit einem Siedepunkt von mindestens 100°C, bevorzugt mindestens 150°C und besonders bevorzugt mindestens 200°C bei Normaldruck (1013 mbar) verstanden.

Überraschende Vorteile können insbesondere bei der Aufreinigung von Methacrylaten, insbesondere von 2-Hydroxyethylmethacrylat, 2,2-Bis(hydroxymethyl)-1,3-propandioldimethacrylat, 2-Hydroxypropylmethacrylat, 3-Hydroxypropylmethacrylat erzielt werden, wobei insbesondere die Aufreinigung von Hydroxyalkylmethacrylaten besonders bevorzugt ist.

Gemäß einem weiteren Aspekt der vorliegenden Erfindung können mit Vorteil Acrylate eingesetzt werden. Zu den Acrylaten, die überraschend einfach mit dem vorliegenden Verfahren aufgereinigt werden können, gehören unter anderem, 2-Hydroxyethylacrylat, 2,2-Bis(hydroxymethyl)-1,3-propandioldiacrylat, 2-Hydroxypropylacrylat, 3-Hydroxypropylacrylat.

Der Anteil an zu reinigendem Monomer in einer erfindungsgemäß einzusetzenden Ausgangszusammensetzung beträgt mindestens 95 Gew.-% und ganz besonders bevorzugt mindestens 98 Gew.-%. Dieser Anteil kann insbesondere durch Gaschromatographie bestimmt werden.

Neben üblichen produktionsbedingten Neben- und Beiprodukten kann die Ausgangszusammensetzung Polymerisationsinhibitoren enthalten. Zu den bevorzugt einzusetzenden Polymerisationsinhibitoren zählen insbesondere Phenolverbindungen, wie zum Beispiel Hydrochinone, Hydrochinonether, wie Hydrochinonmonomethylether, tert-Butylhydrochinon, 2,6-Di-tert-butylhydrochinon, 2,5-Di-tert-butylhydrochinon, 2,4-Dimethyl-6-tert-butylphenol oder Di-tert-butylbrenzcatechin;

p-Phenylendiamine, wie zum Beispiel *N,N'*-Diphenyl-p-phenylendiamin, *N,N'*-Di-2-naphthyl-*p*-phenylendiamin, *N,N'*-Di-

*p*-tolyl-*p*-phenylendiamin, *N*-1,3-Dimethylbutyl-*N'*-phenyl-*p*-phenylendiamin und *N*-1,4-Dimethylpentyl-*N'*-phenyl-*p*-phenylendiamin;

Amine, wie zum Beispiel Thiodiphenylamin und Phenothiazin; Kupferdialkyldithiocarbamate, wie zum Beispiel Kupferdimethyldithiocarbamate, Kupferdiethyldithiocarbamate und Kupferdibutyldithiocarbamate;

Nitrosoverbindungen, wie zum Beispiel Nitrosodiphenylamin, Isoamylnitrit, N-Nitrosocyclohexyihydroxylamin, N-Nitroso-N-phenyl-N-hydroxylamin und deren Salze; und N-Oxylverbindungen, wie zum Beispiel 2,2,4,4-Tetramethylazetidin-1-oxyl, 2,2-Dimethyl-4,4-dipropylazetidin-1-oxyl, 2,2,5,5-Tetramethylpyrrolidin-1-oxyl, 2,2,5,5-Tetramethyl-3-oxopyrrolidin-1-oxyl, 2,2,6,6-Tetramethylpiperidin-1-oxyl, 4-Hydroxy-2,2,6,6-tetramethylpiperidin-1-oxyl, 6-Aza-7,7-dimethyl-spiro[4,5]decan-6-oxyl, 2,2,6,6-Tetramethyl-4-acetoxypiperidin-1-oxyl und 2,2,6,6-Tetramethyl-4-benzoyloxypiperidin-1-oxyl;

Methylenblau, Nigrosin Base BA, 1,4-Benzochinon, sterisch gehinderte Phenole, beispielsweise 2,4-dimethyl-6-tert-butylphenol und/oder Tocopherol-Verbindungen, vorzugsweise α-Tocopherol.

Diese Verbindungen können einzeln oder in Form von Mischungen eingesetzt werden und sind im Allgemeinen kommerziell erhältlich. Für weitere Details wird auf die gängige Fachliteratur, insbesondere auf das Römpp-Lexikon Chemie; Herausgeber: J. Falbe, M. Regitz; Stuttgart, New York; 10. Auflage (1996); Stichwort "Antioxidantien" und die an dieser Stelle zitierten Literaturstellen verwiesen.

Überraschende Vorteile können insbesondere durch den Einsatz von Ausgangszusammensetzungen erzielt werden, die vorzugsweise 1 bis 200 ppm, besonders bevorzugt 5 bis 150 ppm und ganz besonders bevorzugt 10 bis 70 ppm Polymerisationsinhibitor enthalten.

Von besonderem Interesse sind hierbei insbesondere Ausgangszusammensetzungen, die Hydrochinonmonomethylether, 4-Hydroxy-2,2,6,6-tetramethylpiperidin-N-oxyl, Tocopherol, *N,N*-Diethylhydroxylamin, Ammonium-*N*-nitrosophenylhydroxylamin (Cupferron) und/oder Hydrochinon umfassen, ohne dass hierdurch eine Begrenzung der vorliegenden Erfindung erfolgen soll. Überraschende Vorteile zeigen insbesondere Ausgangszusammensetzungen mit einem Gewichtsverhältnis von Hydrochinonmonomethylether zu 4-Hydroxy-2,2,6,6-tetramethylpiperidin-N-oxyl im Bereich von 100:1 bis 1:1, vorzugsweise im Bereich von 40:1 bis 10:1.

Gemäß einem weiteren Aspekt der vorliegenden Erfindung sind Ausgangszusammensetzungen bevorzugt, die höchstens einen geringen Anteil an *N,N'*-Diphenyl-p-phenylendiamin, *N,N'*-Di-2-naphthyl-*p*-phenylendiamin, *N,N'*-Di-p-tolyl-*p*-phenylendiamin, *N*-1,3-Dimethylbutyl-*N'*-phenyl-*p*-phenylendiamin, *N*-1,4-Dimethylpentyl-*N'*-phenyl-*p*-phenylendiamin, Phenothiazin, Nigrosin Base BA und/oder 1,4-Benzochinon aufweisen, wobei deren Anteil vorzugsweise höchstens 10 ppm, besonders bevorzugt höchstens 1 ppm beträgt.

Das Verfahren der vorliegenden Erfindung zeichnet sich insbesondere dadurch aus, dass mindestens ein Teil der Ausgangszusammensetzung in einem Kurzwegverdampfer verdampft und kondensiert wird. Bei einem Kurzwegverdampfer ist der Kondensator innerhalb des Verdampfers angeordnet, so dass das Brüdenrohr entfällt.

Überraschende Vorteile können insbesondere durch die Verwendung von Kurzwegverdampfern erzielt werden, bei denen das Verhältnis von Verdampferfläche zur Kondensatorfläche im Bereich von 0,1 bis 10 liegt.

Gemäß einer besonderen Ausgestaltung kann ein Kurzwegverdampfer eingesetzt werden, der ein Wischsystem umfasst, mit dem die zu verdampfende Ausgangszusammensetzung gleichmäßig über die Verdampferoberfläche verteilt wird. Zu den bevorzugten Systemen gehören Rollenwischer, Klappenwischer, Pendelklappenwischer, Starrflügelwischer und Schab-Kratz-Rotor-Wischer. Hierbei können überraschende Vorteile durch den Einsatz eines Schab-Kratz-Rotors erzielt werden, bei dem das Wischelement mit Federspannkraft gegen die Verdampferoberfläche gedrückt wird.

Mindestens ein Teil der Ausgangszusammensetzung wird gemäß der vorliegenden Erfindung in einem Kurzwegverdampfer verdampft. Hierbei gilt die Relation (I)

$$\dot{m} \leq 1800 \, \frac{kg \cdot \sqrt{K}}{mbar \cdot m^2 \cdot h \cdot \sqrt{\dfrac{kg}{kmol}}} \cdot p_i \cdot \left(\frac{\tilde{M}}{T}\right)^{0,5} \qquad \text{(I)},$$

worin

$\tilde{M}$ die durchschnittliche Molmasse der Brüden im Kurzwegverdampfer in kg/kmol

T die Temperatur der Brüden in K

$p_i$ der Druck im Kurzwegverdampfer in mbar und

$\dot{m}$ die Massenstromdichte der Brüden in kg/(m$^2$·h)

ist.

Dementsprechend werden die Bedingungen so gewählt, dass die Massenstromdichte der Brüden kleiner oder

$$1800 \cdot p_i \cdot \left( \frac{\tilde{M}}{T} \right)^{0,5}$$

gleich dem Produkt aus                    ist, falls die Einheiten aus Gründen der Übersichtlichkeit nicht aufgeführt werden. Vorzugsweise ist die Massenstromdichte der Brüden kleiner oder gleich dem Produkt aus

$$1700 \cdot p_i \cdot \left( \frac{\tilde{M}}{T} \right)^{0,5},$$

wobei die Variablen die zuvor dargelegte Bedeutung haben.

Eine hohe Massenstromdichte der Brüden führt zu einem sehr wirtschaftlichen Verfahren. Bei einem Überschreiten der zuvor dargelegten Relation wird die Reinheit und die Qualität des Produkts nachteilig beeinflusst. Weiterhin trägt ein Ausgangsprodukt mit einem hohen Anteil an Monomer überraschend zu einer unerwarteten Qualitätsverbesserung bei.

Der Begriff "Brüden" bezeichnet in obiger Relation die Gase, die im Verdampfer verdampft und im Kondensator kondensiert werden. Bei Vernachlässigung von Verunreinigungen bezeichnet dieser Begriff die verdampften und kondensierten Monomere.

Die durchschnittliche Molmasse der Brüden im Kurzwegverdampfer ergibt sich aus der Molmasse der Komponenten des Kondensats. Die durchschnittliche Molmasse des Kondensats kann durch Analyse der Bestandteile des Kondensats ermittelt werden, welche beispielsweise durch Gaschromatographie erfolgen kann. Hierbei bezieht sich der Durchschnitt auf das Zahlenmittel des Molekulargewichts.

Die Temperatur der Brüden in Relation (I) bezieht sich auf die Temperatur zwischen der Verdampfungsfläche und der Kondensationsfläche des Kurzwegverdampfers. Diese Temperatur kann mit einem Temperaturfühler, insbesondere einem Thermoelement oder einem Widerstandstemperaturfühler gemäß DIN IEC 60751 bzw. DIN 43772 bestimmt werden. Die Temperatur der Brüden bzw. des Gases kann insbesondere über die Regelung des Druckes sowie der Teilchenanzahl im Verdampfer eingestellt werden.

Der Druck bezieht sich auf den Druck in dem Kurzwegverdampfer und kann an der Stelle bestimmt werden, an der das Vakuum im Kurzwegverdampfer gebildet wird.

Der Druck, bei dem die Verdampfung erfolgt, liegt im Bereich von 10$^{-5}$ mbar bis 10 mbar absolut, besonders bevorzugt im Bereich von 10$^{-4}$ mbar bis 1 mbar absolut.

Die Massenstromdichte der Brüden $\dot{m}$ ergibt sich aus der Formel (II)

$$\dot{m} = \frac{\dot{M}}{A} \qquad \qquad \text{(II)},$$

worin

$\dot{M}$    die verdampfte Menge in kg/h und
A    die Verdampferoberfläche in m$^2$ ist.

Die verdampfte Menge kann über die Menge an Brüdenkondensat berechnet werden, die über eine Zeit von 1 Stunde gebildet wird. Die Verdampferoberfläche bezieht sich hierbei auf die beheizte innere Oberfläche über die im Kurzwegverdampfer eine Verdampfung der Ausgangszusammensetzung erfolgt.

Die obere Grenze der Massenstromdichte ergibt sich aus obiger Relation (I). Die untere Grenze ergibt sich insbesondere aus der Effizienz des Verfahrens.

Die Massenstromdichte kann insbesondere über die dem Kurzwegverdampfer zugeführte Verdampfungsenergie, insbesondere die Temperatur des Heizmittels, und der bei der Kondensation des Dampfes abgeführten Wärmemenge, insbesondere die Temperatur des Kühlmittels, gesteuert werden.

Vorzugsweise wird die Verdampfung bei einer Temperatur im Bereich von 15°C bis 150°C, besonders bevorzugt im Bereich von 20 bis 110°C und ganz besonders bevorzugt 25°C bis 60°C durchgeführt, wobei diese Angaben auf die mittlere Temperatur des Heizmediums bezogen sind.

Gemäß einem besonderen Aspekt kann die Kondensation bei einer Temperatur im Bereich von -50°C bis 60°C, besonders bevorzugt im Bereich von -25°C bis 50°C und ganz besonders bevorzugt -7°C bis 35°C erfolgen, wobei diese Angaben auf die mittlere Temperatur des Kühlmediums bezogen sind.

Die Differenz zwischen der Verdampfungstemperatur und der Kondensationstemperatur kann vorzugsweise im Bereich von 1 bis 180°C, besonders bevorzugt 2°C bis 100°C und ganz besonders bevorzugt 5 bis 60°C liegen.

Das vorliegende Aufreinigungsverfahren kann bevorzugt kontinuierlich durchgeführt werden, wobei die mittlere Verweilzeit des Monomeren insbesondere im Bereich von 1 Sekunde bis 5 Minuten, besonders bevorzugt im Bereich von 5 Sekunden bis 3 Minuten liegen kann.

Die Ausgangszusammensetzung umfasst bei der Verdampfung der aufzureinigenden Monomere höchstens 5 Gew.-% und ganz besonders bevorzugt höchstens 2 Gew.-% an Verbindungen mit einem Siedepunkt der mindestens 5°C unterhalb des Siedepunkts des aufzureinigenden Monomeren liegt. Dementsprechend können der Ausgangszusammensetzung Komponenten mit einem niedrigen Siedepunkt entzogen werden, bevor die in der Ausgangszusammensetzung enthaltenen Monomere verdampft und kondensiert werden.

Diese Komponenten mit einem niedrigen Siedepunkt können insbesondere durch Verdampfung aus der Ausgangszusammensetzung entfernt werden. Vorzugsweise kann die Abtrennung der Komponenten mit einem niedrigen Siedepunkt in einem Kurzwegverdampfer durchgeführt werden.

Der Druck, bei dem die Komponenten mit einem niedrigen Siedepunkt abgetrennt werden, liegt vorzugsweise in einem Bereich von 1 mbar bis 20 mbar, besonders bevorzugt im Bereich von 2 mbar bis 10 mbar. Vorzugsweise liegt die Temperatur, bei der die Komponenten mit einem niedrigen Siedepunkt abgetrennt werden, im Bereich von 40°C bis 150°C, besonders bevorzugt im Bereich von 50°C bis 110°C, wobei diese Angaben auf die mittlere Temperatur des Heizmediums bezogen sind.

Gemäß einer besonderen Fortentwicklung kann der nach der Verdampfung mindestens eines Teils der Monomeren aus der Ausgangszusammensetzung erhaltene Rückstand aufgearbeitet werden, um Monomerreste aus diesem Rückstand abzutrennen. Dies kann insbesondere durch Verdampfung mindestens eines Teils dieses Rückstands in einem Kurzwegverdampfer erfolgen. Der Druck, bei dem die Verdampfung eines Teils des Rückstands erfolgt, liegt vorzugsweise in einem Bereich von $10^{-5}$ mbar bis 10 mbar absolut, besonders bevorzugt in einem Bereich von $10^{-4}$ mbar bis 1 mbar. Vorzugsweise kann die Verdampfung des Rückstands bei einer Temperatur im Bereich von 15°C bis 150°C, besonders bevorzugt im Bereich von 20 bis 110°C und ganz besonders bevorzugt 25°C bis 60°C durchgeführt werden.

Die aus dem Rückstand gewonnenen Monomere werden vorzugsweise nochmals durch Verdampfung aufgereinigt. Hierzu kann das aus dem Rückstand erhaltene Kondensat der Ausgangszusammensetzung zugemischt werden, wobei dies vor oder nach der Abtrennung von Komponenten mit einem niedrigen Siedepunkt erfolgen kann.

Durch das vorliegende Verfahren sind überraschend hohe Produktqualitäten möglich. So kann das aufgereinigte Produkt mindestens 98 Gew.-%, besonders bevorzugt mindestens 99 Gew.-% und ganz besonders bevorzugt 99,5 Gew.-% Monomer umfassen.

Gemäß einem besonderen Aspekt der vorliegenden Erfindung können die durch das Verfahren erhältlichen Monomere eine geringe Farbzahl aufweisen. So beträgt die Farbzahl nach einer erfindungsgemäßen Aufreinigung höchstens 20, besonders bevorzugt höchstens 10 und ganz besonders bevorzugt höchstens 5. Die Farbzahl kann insbesondere durch das in DE-A-10 131 479 dargelegte Verfahren (Bestimmung der Farbe nach der Platin-Cobalt-Skala; auch APHA oder Trübungszahl genannt) bestimmt werden.

Überraschend kann durch die vorliegende Erfindung eine Monomerzusammensetzung bereitgestellt werden, deren Farbzahl auch nach einer Lagerung bei 30°C über einen Zeitraum von 6 Monaten um höchstens 5 Einheiten zunimmt.

Gemäß einem weiteren Aspekt der vorliegenden Erfindung kann die Ausgangszusammensetzung mehrfach, insbesondere zweimal, dreimal, viermal oder häufiger mit einem Kurzwegverdampfer aufgereinigt werden, wobei mindestens ein Teil des jeweiligen Rückstands ein weiteres Mal verdampft und kondensiert wird.

Darüber hinaus sind auch Ausführungsformen des vorliegenden Verfahrens von Interesse, bei denen die Ausgangszusammensetzung mehrfach, insbesondere zweimal, dreimal, viermal oder häufiger mit einem Kurzwegverdampfer aufgereinigt wird, wobei mindestens ein Teil eines oder mehrerer Kondensate ein weiteres Mal verdampft und kondensiert wird.

Nachfolgend soll die vorliegende Erfindung anhand von Beispielen und eines Vergleichsbeispiels näher erläutert werden, ohne dass hierdurch eine Beschränkung derselben erfolgen soll.

Beispiel 1

In einem Kurzwegverdampfer wurde eine Zusammensetzung eingeleitet, die ca. 98 Gew.-% 2-Hydroxyethylmethacrylat (HEMA) und ca. 50 ppm Hydrochinonmonomethylether umfasste. Der Druck im Kurzwegverdampfer und die Temperatur der Brüden wurden so eingestellt, dass gemäß Relation (I) ein Wert von 440 kg/h/m² erhalten wurde. Die durch die Verdampfungstemperatur und die Kondensationstemperatur eingestellte Massenstromdichte der Brüden

EP 2 427 421 B1

betrug 27 kg/h/m². Bei diesen Bedingungen wurden Verunreinigungen mit einem geringen Siedepunkt durch Verdampfung abgetrennt, wobei das aufzureinigende 2-Hydroxyethylmethacrylat im Rückstand verblieb. Im Kurzwegverdampfer wurde keine wesentliche Polymerisation der zugeführten Zusammensetzung festgestellt.

Der aus dem ersten Verdampfungsprozess erhaltene Rückstand wurde ein zweites Mal in einen Kurzwegverdampfer eingeleitet. Hierbei wurden der Druck im Kurzwegverdampfer und die Temperatur der Brüden so eingestellt, dass gemäß Relation (I) ein Wert von 112 kg/h/m² erhalten wurde. Die durch die Verdampfungstemperatur und die Kondensationstemperatur eingestellte Massenstromdichte der Brüden betrug 56 kg/h/m². In diesem Verdampfungsschritt wurde 2-Hydroxyethylmethacrylat verdampft. Die bei diesem Schritt kondensierte Zusammensetzung enthielt ca. 99,5 Gew.-% 2-Hydroxyethylmethacrylat. Im Kurzwegverdampfer wurde keine wesentliche Polymerisation der zugeführten Zusammensetzung festgestellt.

Beispiel 2

In einem Kurzwegverdampfer wurde eine Zusammensetzung eingeleitet, die ca. 98 Gew.-% 2-Hydroxyethylmethacrylat (HEMA) und ca. 50 ppm Hydrochinonmonomethylether umfasste. Der Druck im Kurzwegverdampfer und die Temperatur der Brüden wurden so eingestellt, dass gemäß Relation (I) ein Wert von 369 kg/h/m² erhalten wurde. Die durch die Verdampfungstemperatur und die Kondensationstemperatur eingestellte Massenstromdichte der Brüden betrug 32 kg/h/m². Bei diesen Bedingungen wurden Verunreinigungen mit einem geringen Siedepunkt durch Verdampfung abgetrennt, wobei das aufzureinigende 2-Hydroxyethylmethacrylat im Rückstand verblieb. Im Kurzwegverdampfer wurde keine wesentliche Polymerisation der zugeführten Zusammensetzung festgestellt.

Der aus dem ersten Verdampfungsprozess erhaltene Rückstand wurde ein zweites Mal in einen Kurzwegverdampfer eingeleitet. Hierbei wurden der Druck im Kurzwegverdampfer und die Temperatur der Brüden so eingestellt, dass gemäß Relation (I) ein Wert von 296 kg/h/m² erhalten wurde. Die durch die Verdampfungstemperatur und die Kondensationstemperatur eingestellte Massenstromdichte der Brüden betrug 30 kg/h/m². In diesem Verdampfungsschritt wurde 2-Hydroxyethylmethacrylat verdampft. Die bei diesem Schritt kondensierte Zusammensetzung enthielt ca. 99,5 Gew.-% 2-Hydroxyethylmethacrylat. Im Kurzwegverdampfer wurde keine wesentliche Polymerisation der zugeführten Zusammensetzung festgestellt.

Vergleichsbeispiel 1

In einem Kurzwegverdampfer wurde eine Zusammensetzung eingeleitet, die ca. 98 Gew.-% 2-Hydroxyethylmethacrylat (HEMA) und ca. 50 ppm Hydrochinonmonomethylether umfasste. Der Druck im Kurzwegverdampfer und die Temperatur der Brüden wurden so eingestellt, dass gemäß Relation (I) ein Wert von 229 kg/h/m² erhalten wurde. Die durch die Verdampfungstemperatur und die Kondensationstemperatur eingestellte Massenstromdichte der Brüden betrug 27 kg/h/m². Bei diesen Bedingungen wurden Verunreinigungen mit einem geringen Siedepunkt durch Verdampfung abgetrennt, wobei das aufzureinigende 2-Hydroxyethylmethacrylat im Rückstand verblieb. Im Kurzwegverdampfer wurde keine wesentliche Polymerisation der zugeführten Zusammensetzung festgestellt.

Der aus dem ersten Verdampfungsprozess erhaltene Rückstand wurde ein zweites Mal in einen Kurzwegverdampfer eingeleitet. Hierbei wurden der Druck im Kurzwegverdampfer und die Temperatur der Brüden so eingestellt, dass gemäß Relation (I) ein Wert von 63 kg/h/m² erhalten wurde. Die durch die Verdampfungstemperatur und die Kondensationstemperatur eingestellte Massenstromdichte der Brüden betrug 81 kg/h/m². In diesem Verdampfungsschritt wurde 2-Hydroxyethylmethacrylat verdampft. Die bei diesem Schritt kondensierte Zusammensetzung enthielt ca. 98 Gew.-% 2-Hydroxyethylmethacrylat. Im Kurzwegverdampfer wurde keine wesentliche Polymerisation der zugeführten Zusammensetzung festgestellt.

Das Vergleichsbeispiel 1 zeigt deutlich, dass ein Arbeiten außerhalb der in Relation (I) angegebenen Werte zu keiner Verbesserung der Produktreinheit führte, wobei die Ausgangszusammensetzung bereits eine relativ hohe Reinheit aufwies. Hierbei ist zu bedenken, dass Kurzwegverdampfer üblich nicht zur Feinreinigung, sondern zur Aufarbeitung von Rückständen eingesetzt werden.

**Patentansprüche**

1. Verfahren zur Aufreinigung von Monomeren, wobei die Monomere (Meth)acrylate mit einer Hydroxygruppe im Alkylrest sind, wobei mindestens ein Teil der in einer Ausgangszusammensetzung enthaltenen Monomere verdampft und anschließend kondensiert wird, **dadurch gekennzeichnet, dass** mindestens ein Teil der Ausgangszusammensetzung in einem Kurzwegverdampfer verdampft wird, wobei die Massenstromdichte der Brüden $\dot{m}$ gemäß der Relation (I)

$$\dot{m} \leq 1800 \frac{kg \cdot \sqrt{K}}{mbar \cdot m^2 \cdot h \cdot \sqrt{\dfrac{kg}{kmol}}} \cdot p_i \cdot \left(\frac{\tilde{M}}{T}\right)^{0,5} \qquad \text{(I)},$$

worin

$\tilde{M}$ die durchschnittliche Molmasse der Brüden im Kurzwegverdampfer in kg/kmol
T Temperatur der Brüden in K
$p_i$ Druck im Kurzwegverdampfer in mbar
$\dot{m}$ die Massenstromdichte der Brüden in kg/(m$^2$·h)

ist, gewählt wird und
die Ausgangszusammensetzung mindestens 95 Gew.-% Monomer umfasst.

2. Verfahren gemäß Anspruch 1, **dadurch gekennzeichnet, dass** die Verdampfung bei einer Temperatur im Bereich von 15°C bis 150°C durchgeführt wird.

3. Verfahren gemäß mindestens einem der vorhergehenden Ansprüche, **dadurch gekennzeichnet, dass** die Ausgangszusammensetzung ein hochsiedendes Monomer umfasst.

4. Verfahren gemäß Anspruch 1, **dadurch gekennzeichnet, dass** das Methacrylat ausgewählt ist aus 2-Hydroxyethylmethacrylat, 2-Hydroxypropylmethacrylat, 3-Hydroxypropylmethacrylat oder 2,3-Dihydroxypropylmethacrylat.

5. Verfahren gemäß Anspruch 1, **dadurch gekennzeichnet, dass** das Acrylat ausgewählt ist aus 2-Hydroxyethylacrylat, 2-Hydroxypropylacrylat oder 3-Hydroxypropyl acrylat.

6. Verfahren gemäß mindestens einem der vorhergehenden Ansprüche, **dadurch gekennzeichnet, dass** die Verweilzeit des Monomers im Bereich von 1 Sekunde bis 5 Minuten liegt.

7. Verfahren gemäß mindestens einem der vorhergehenden Ansprüche, **dadurch gekennzeichnet, dass** die Ausgangszusammensetzung mindestens einen Polymerisationinhibitor umfasst.

8. Verfahren gemäß Anspruch 7, **dadurch gekennzeichnet, dass** die Ausgangszusammensetzung 1 bis 200 ppm Polymerisationinhibitor umfasst.

9. Verfahren gemäß mindestens einem der vorhergehenden Ansprüche, **dadurch gekennzeichnet, dass** der Ausgangszusammensetzung Komponenten mit einem niedrigen Siedepunkt entzogen werden, bevor die in der Ausgangszusammensetzung enthaltenen Monomere verdampft und kondensiert werden.

10. Verfahren gemäß Anspruch 9, **dadurch gekennzeichnet, dass** die Komponenten mit einem niedrigen Siedepunkt durch Verdampfung in einem Kurzwegverdampfer der Ausgangszusammensetzung entzogen werden.

11. Verfahren gemäß Anspruch 10, **dadurch gekennzeichnet, dass** die Verdampfung zur Entfernung der Komponenten mit einem niedrigen Siedepunkt bei einer Temperatur im Bereich von 40°C bis 150°C durchgeführt wird.

12. Verfahren gemäß mindestens einem der vorhergehenden Ansprüche, **dadurch gekennzeichnet, dass** der nach der Verdampfung des Monomeren in einem Kurzwegverdampfer erhaltene Rückstand einer Verdampfung und Kondensation in einem Kurzwegverdampfer unterzogen wird.

13. Verfahren gemäß Anspruch 12, **dadurch gekennzeichnet, dass** die Verdampfung des Rückstands bei einer Temperatur im Bereich von 15°C bis 150°C durchgeführt wird.

14. Verfahren gemäß mindestens einem der vorhergehenden Ansprüche, **dadurch gekennzeichnet, dass** das aufgereinigte Produkt mindestens 99 Gew.-% Monomer umfasst.

**Claims**

1. Process for purifying monomers, the monomers being (meth)acrylates having a hydroxyl group in the alkyl radical, by evaporating at least a portion of the monomers present in a starting composition and then condensing it, **characterized in that** at least a portion of the starting composition is evaporated in a short-path evaporator, the mass flow density of the vapours $\dot{m}$ being selected according to the relation (I)

$$\dot{m} \leq 1800 \frac{kg \cdot \sqrt{K}}{mbar \cdot m^2 \cdot h \cdot \sqrt{\dfrac{kg}{kmol}}} \cdot p_i \cdot \left( \frac{\widetilde{M}}{T} \right)^{0.5} \qquad (\mathrm{I})$$

in which

$\widetilde{M}$ is the average molar mass of the vapours in the short-path evaporator in kg/kmol
T is the temperature of the vapours in K
$p_i$ is the pressure in the short-path evaporator in mbar
$\dot{m}$ is the mass flow density of the vapours in kg/(m$^2$·h),

and
the starting composition comprises at least 95% by weight of monomer.

2. Process according to Claim 1, **characterized in that** the evaporation is performed at a temperature in the range from 15°C to 150°C.

3. Process according to at least one of the preceding claims, **characterized in that** the starting composition comprises a high-boiling monomer.

4. Process according to Claim 1, **characterized in that** the methacrylate is selected from 2-hydroxyethyl methacrylate, 2-hydroxypropyl methacrylate, 3-hydroxypropyl methacrylate and 2,3-dihydroxypropyl methacrylate.

5. Process according to Claim 1, **characterized in that** the acrylate is selected from 2-hydroxyethyl acrylate, 2-hydroxypropyl acrylate and 3-hydroxypropyl acrylate.

6. Process according to at least one of the preceding claims, **characterized in that** the residence time of the monomer is in the range from 1 second to 5 minutes.

7. Process according to at least one of the preceding claims, **characterized in that** the starting composition comprises at least one polymerization inhibitor.

8. Process according to Claim 7, **characterized in that** the starting composition comprises 1 to 200 ppm of polymerization inhibitor.

9. Process according to at least one of the preceding claims, **characterized in that** components with a low boiling point are withdrawn from the starting composition before the monomers present in the starting composition are evaporated and condensed.

10. Process according to Claim 9, **characterized in that** the components with a low boiling point are withdrawn from the starting composition by evaporation in a short-path evaporator.

11. Process according to Claim 10, **characterized in that** the evaporation to remove the components with a low boiling point is performed at a temperature in the range from 40°C to 150°C.

12. Process according to at least one of the preceding claims, **characterized in that** the residue obtained after the evaporation of the monomer in a short-path evaporator is subjected to an evaporation and condensation in a short-path evaporator.

**13.** Process according to Claim 12, **characterized in that** the evaporation of the residue is performed at a temperature in the range from 15°C to 150°C.

**14.** Process according to at least one of the preceding claims, **characterized in that** the purified product comprises at least 99% by weight of monomer.

**Revendications**

**1.** Procédé de purification de monomères, les monomères étant des (méth)acrylates contenant un groupe hydroxyle dans le radical alkyle, selon lequel au moins une partie des monomères contenus dans une composition de départ est évaporée, puis condensée, **caractérisé en ce qu'**au moins une partie de la composition de départ est évaporée dans un évaporateur à court trajet, la densité de flux massique des vapeurs $\dot{m}$ étant choisie selon la relation (I) :

$$\dot{m} \leq 1800 \frac{kg \cdot \sqrt{K}}{mbar \cdot m^2 \cdot h \cdot \sqrt{\dfrac{kg}{kmol}}} \cdot p_i \cdot \left( \frac{\tilde{M}}{T} \right)^{0,5} \quad \text{(I)},$$

dans laquelle

$\tilde{M}$ est la masse molaire moyenne des vapeurs dans l'évaporateur à court trajet en kg/kmol,
T est la température des vapeurs en K,
$p_i$ est la pression dans l'évaporateur à court trajet en mbar,
$\dot{m}$ est la densité de flux massique des vapeurs en kg/(m²·h), et

la composition de départ comprend au moins 95 % en poids de monomères.

**2.** Procédé selon la revendication 1, **caractérisé en ce que** l'évaporation est réalisée à une température dans la plage allant de 15 °C à 150 °C.

**3.** Procédé selon au moins l'une quelconque des revendications précédentes, **caractérisé en ce que** la composition de départ comprend un monomère de point d'ébullition élevé.

**4.** Procédé selon la revendication 1, **caractérisé en ce que** le méthacrylate est choisi parmi le méthacrylate de 2-hydroxyéthyle, le méthacrylate de 2-hydroxypropyle, le méthacrylate de 3-hydroxypropyle ou le méthacrylate de 2,3-dihydroxypropyle.

**5.** Procédé selon la revendication 1, **caractérisé en ce que** l'acrylate est choisi parmi l'acrylate de 2-hydroxyéthyle, l'acrylate de 2-hydroxypropyle ou l'acrylate de 3-hydroxypropyle.

**6.** Procédé selon au moins l'une quelconque des revendications précédentes, **caractérisé en ce que** le temps de séjour du monomère se situe dans la plage allant de 1 seconde à 5 minutes.

**7.** Procédé selon au moins l'une quelconque des revendications précédentes, **caractérisé en ce que** la composition de départ comprend au moins un inhibiteur de polymérisation.

**8.** Procédé selon la revendication 7, **caractérisé en ce que** la composition de départ comprend 1 à 200 ppm d'inhibiteur de polymérisation.

**9.** Procédé selon au moins l'une quelconque des revendications précédentes, **caractérisé en ce que** des composants ayant un point d'ébullition bas sont extraits de la composition de départ avant l'évaporation et la condensation des monomères contenus dans la composition de départ.

**10.** Procédé selon la revendication 9, **caractérisé en ce que** les composants ayant un point d'ébullition bas sont extraits de la composition de départ par évaporation dans un évaporateur à court trajet.

**11.** Procédé selon la revendication 10, **caractérisé en ce que** l'évaporation pour l'élimination des composants ayant un point d'ébullition bas est réalisée à une température dans la plage allant de 40 °C à 150 °C.

**12.** Procédé selon au moins l'une quelconque des revendications précédentes, **caractérisé en ce que** le résidu obtenu après l'évaporation des monomères dans un évaporateur à court trajet est soumis à une évaporation et une condensation dans un évaporateur à court trajet.

**13.** Procédé selon la revendication 12, **caractérisé en ce que** l'évaporation du résidu est réalisée à une température dans la plage allant de 15 °C à 150 °C.

**14.** Procédé selon au moins l'une quelconque des revendications précédentes, **caractérisé en ce que** le produit purifié comprend au moins 99 % en poids de monomères.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1090904 A
- US 4375534 A
- EP 0365777 A
- JP 2008297242 B
- DE 10131479 A

**In der Beschreibung aufgeführte Nicht-Patentliteratur**

- Römpp-Lexikon Chemie. 1996